(19) Europäisches Patentamt
European Patent Office
Office européen des brevets

(11) Veröffentlichungsnummer: **0 007 501**
**A1**

(12) # EUROPÄISCHE PATENTANMELDUNG

(21) Anmeldenummer: 79102329.4

(22) Anmeldetag: 09.07.79

(51) Int. Cl.³: C 07 F 9/65
A 01 N 57/08, A 01 N 57/16
A 01 N 57/32

(30) Priorität: 20.07.78 DE 2831852

(43) Veröffentlichungstag der Anmeldung:
06.02.80 Patentblatt 80/3

(84) Benannte Vertragsstaaten:
AT BE CH DE FR GB IT NL SE

(71) Anmelder: BAYER Aktiengesellschaft
Zentralbereich Patente,Marken und Lizenzen Bayerwe
D-5090 Leverkusen 1(DE)

(72) Erfinder: Maurer, Fritz, Dr.
Roeberstrasse 8
D-5600 Wuppertal 1(DE)

(72) Erfinder: Schröder, Rolf, Dr.
Pahlkestrasse 17
D-5600 Wuppertal 1(DE)

(72) Erfinder: Hammann, Ingeborg, Dr.
Belfortstrasse 9
D-5000 Köln 1(DE)

(72) Erfinder: Behrenz, Wolfgang, Dr.
Untergründemich 14
D-5063 Overath(DE)

(72) Erfinder: Homeyer, Bernhard, Dr.
Obere Strasse 28
D-5090 Leverkusen 3(DE)

(72) Erfinder: Stendel, Wilhelm, Dr.
In den Birken 55
D-5600 Wuppertal 1(DE)

(54) **Cyclopropyl-pyrimidin-(4)yl-(thiono)(thiol)-phosphor (phospon)-säureester bzw. -esteramide, Verfahren zu ihrer Herstellung, Schädlingsbekämpfungsmittel, Verfahren zu ihrer Herstellung, ihre Verwendung, Verfahren zur Bekämpfung von Schädlingen.**

(57) Die Erfindung betrifft neue 2-Cyclopropyl- pyrimidin(4)yl-(thiono)-(thiol)- phosphor(phosphon)- säureester bzw. -esteramide der Formel

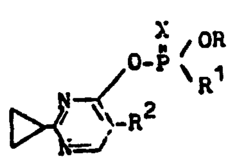

in welcher
R für Alkyl steht,
R¹ für Alkyl, Alkoxy, Alkylthio, Alkylamino oder Phenyl steht,
R² für Wasserstoff, Alkyl oder Halogen steht und
X für Sauerstoff oder Schwefel steht.

Die neuen Verbindungen zeichnen sich durch hohe Wirksamkeit als Schädlingsbekämpfungsmittel, insbesondere durch hohe insektizide und akarizide Wirksamkeit aus, ebenso durch nematizide Wirksamkeit.

EP 0 007 501 A1

BAYER AKTIENGESELLSCHAFT 5090 Leverkusen, Bayerwerk
Zentralbereich
Patente, Marken und Lizenzen
Ad-kl
Typ Ia

BEZEICHNUNG GEÄNDERT
siehe Titelseite

2-Cyclopropyl-pyrimidin(4)yl-(thiono)(thiol)-phosphor-(phosphon)-säureester bzw. -esteramide, Verfahren zu ihrer Herstellung und ihre Verwendung als Schädlingsbekämpfungsmittel

Die Erfindung betrifft neue 2-Cyclopropyl-pyrimidin(4)-yl-(thiono)(thiol)-phosphor(phosphon)-säureester bzw. -esteramide, ein Verfahren zu ihrer Herstellung sowie ihre Verwendung als Schädlingsbekämpfungsmittel, insbesondere als Insektizide, Akarizide und Nematizide.

Es ist bekannt, daß bestimmte Pyrimidinyl-thiono(thiol)-phosphorsäureester, wie z.B. O,O-Diäthyl-O-(2-iso-propyl-6-methyl-pyrimidin(4)yl)-thionophosphorsäureester, O-Äthyl-S-n-propyl-O-(2-iso-propyl-6-methyl-pyrimidin-(4)yl)-thiono-thiol-phosphorsäureester und O,O-Diäthyl-O-(2-methylthio-6-methyl-pyrimidin(4)yl)-thionophosphorsäureester, insektizid, akarizid und nematizid wirksam sind (vgl. DE-PS 910 652 und DE-OS 2 360 877).

Die Wirkung dieser Verbindungen ist jedoch, insbesondere bei niedrigen Aufwandmengen und Konzentrationen, nicht immer zufriedenstellend.

Es wurden nun die neuen 2-Cyclopropyl-pyrimidin(4)yl-(thiono)-(thiol)-phosphor(phosphon)-säureester bzw. -esteramide der Formel

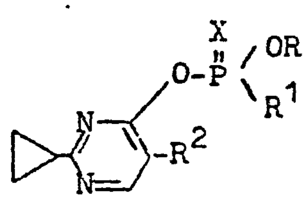

(I)

in welcher

R für Alkyl steht,

$R^1$ für Alkyl, Alkoxy, Alkylthio, Alkylamino oder Phenyl steht,

$R^2$ für Wasserstoff, Alkyl oder Halogen steht und

X für Sauerstoff oder Schwefel steht,

gefunden.

Die neuen Verbindungen zeichnen sich durch hohe Wirksamkeit als Schädlingsbekämpfungsmittel, insbesondere durch hohe insektizide und akarizide Wirksamkeit aus, ebenso durch nematizide Wirksamkeit.

Weiter wurde gefunden, daß man die neuen 2-Cyclopropyl-pyrimidin(4)yl-(thiono)-(thiol)-phosphor(phosphon)-säureester bzw. -esteramide der Formel (I) erhält, wenn man (Thiono)(Thiol)Phosphor(phosphon)-säureesterhalogenide bzw. (Thiono)Phosphorsäureesteramidhalogenide der Formel

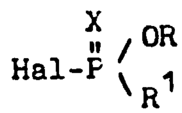

(II)

in welcher

R, $R^1$ und X die oben angegebene Bedeutung haben und

Hal für Chlor oder Brom steht,

mit 2-Cyclopropyl-4-hydroxy-pyrimidinen der Formel

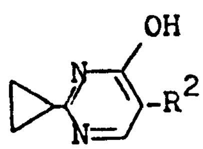

(III)

in welcher

$R^2$ die oben angegebene Bedeutung hat,

gegebenenfalls in Gegenwart eines Säureakzeptors und gegebenenfalls in Gegenwart eines Verdünnungsmittels umsetzt.

Überraschenderweise zeigen die erfindungsgemäßen 2-Cyclopropyl-pyrimidin(4)yl-(thiono)-(thiol)-phosphor-(phosphon)-säureester bzw. -esteramide eine bessere Wirkung als Schädlingsbekämpfungsmittel, insbesondere eine bessere insektizide, nematizide und akarizide Wirkung als die entsprechenden aus dem Stand der Technik bekannten Verbindungen



BAD ORIGINAL

analoger Konstitution und gleicher Wirkungsrichtung. Die Produkte gemäß vorliegender Erfindung stellen somit eine wertvolle Bereicherung der Technik dar.

Verwendet man beispielsweise O-Methyl-äthanphosphonsäureesterchlorid und 2-Cyclopropyl-5-n-propyl-4-hydroxy-pyrimidin als Ausgangsstoffe, so kann die Reaktion dieser Verbindungen durch folgendes Formelschema skizziert werden:

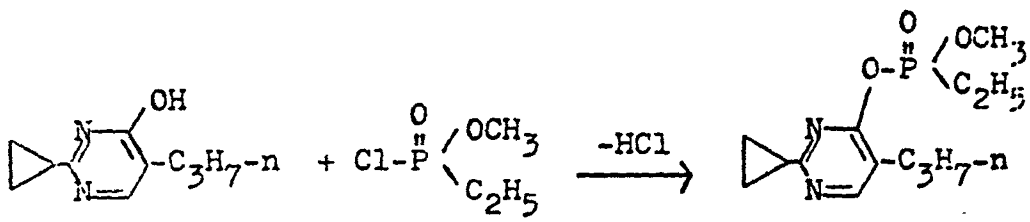


Die als Ausgangsstoffe zu verwendenden (Thiono)(Thiol)-Phosphor(phosphon)-säureesterhalogenide bzw. (Thiono)-Phosphorsäureesteramidhalogenide sind durch Formel (II) definiert. Vorzugsweise stehen darin

R für geradkettiges oder verzweigtes Alkyl mit 1 bis 5, insbesondere mit 1 bis 3 Kohlenstoffatomen,

$R^1$ für geradkettiges oder verzweigtes Alkyl mit 1 bis 5, insbesondere mit 1 bis 3 Kohlenstoffatomen, geradkettiges oder verzweigtes Alkoxy, Alkylthio oder Alkylamino mit 1 bis 5, insbesondere mit 1 bis 3 Kohlenstoffatomen je Alkylrest oder für Phenyl,

X für Sauerstoff oder Schwefel und



BAD ORIGINAL

Hal für Chlor.

Als Beispiele für die Ausgangsstoffe der Formel (II) seien im einzelnen genannt:
O-Methyl-, O-Äthyl-, O-n-Propyl- und O-iso-Propyl-methan-, -äthan-, -propan- und -phenylphosphonsäureesterchlorid sowie die entsprechenden Thionoanalogen, ferner
O,O-Dimethyl-, O,O-Diäthyl-, O,O-Di-n-propyl-, O-Methyl-O-äthyl-, O-Methyl-O-n-propyl-, O-Methyl-O-iso-propyl-, O-Äthyl-O-n-propyl- und O-Äthyl-O-iso-propyl-phosphorsäurediesterchlorid sowie die entsprechenden Thionoanalogen, ferner
O,S-Dimethyl-, O,S-Diäthyl-, O,S-Di-n-propyl-, O,S-Di-iso-propyl-, O-Methyl-S-äthyl-, O-Methyl-S-n-propyl-, O-Methyl-S-iso-propyl-, O-Äthyl-S-methyl-, O-Äthyl-S-n-propyl-, O-Äthyl-S-iso-propyl-, O-n-Propyl-S-methyl-, O-n-Propyl-S-äthyl-, O-n-Propyl-S-iso-propyl-, O-iso-Propyl-S-methyl-, O-iso-Propyl-S-äthyl- und O-iso-Propyl-S-n-propyl-thionophosphorsäurediesterchlorid, sowie die entsprechenden Thionoanalogen, ferner
O-Methyl-N-methyl-, O-Methyl-N-äthyl-, O-Methyl-N-n-propyl-, O-Methyl-N-iso-propyl-, O-Äthyl-N-methyl-, O-Äthyl-N-äthyl-, O-Äthyl-N-n-propyl-, O-Äthyl-N-iso-propyl-, O-Propyl-N-methyl-, O-n-Propyl-N-äthyl-, O-n-Propyl-N-n-propyl-, O-n-Propyl-N-iso-propyl-, O-iso-Propyl-N-methyl-, O-iso-Propyl-N-äthyl-, O-iso-Propyl-N-n-propyl- und O-iso-Propyl-N-iso-propyl-phosphorsäureesteramidchlorid sowie die entsprechenden Thionoanalogen.



BAD ORIGINAL

Die Ausgangsverbindungen der Formel (II) sind bekannt. Die weiter als Ausgangsstoffe zu verwendenden 2-Cyclopropyl-4-hydroxy-pyrimidine sind durch Formel (III) definiert. Vorzugsweise steht darin

$R^2$ für Wasserstoff, geradkettiges oder verzweigtes Alkyl mit 1 bis 5, insbesondere mit 1 bis 3 Kohlenstoffatomen sowie für Chlor oder Brom.

Als Beispiele für die Ausgangsverbindungen der Formel (III) seien im einzelnen genannt:
2-Cyclopropyl-4-hydroxy-pyrimidin,
2-Cyclopropyl-5-methyl-4-hydroxy-pyrimidin,
2-Cyclopropyl-5-äthyl-4-hydroxy-pyrimidin,
2-Cyclopropyl-5-n-propyl-4-hydroxy-pyrimidin,
2-Cyclopropyl-5-iso-propyl-4-hydroxy-pyrimidin,
2-Cyclopropyl-5-chlor-4-hydroxy-pyrimidin und
2-Cyclopropyl-5-brom-4-hydroxy-pyrimidin.

Die Ausgangsverbindungen der Formel (III) sind neu. Verbindungen der Formel (III), in welcher $R^2$ für Wasserstoff oder Alkyl steht, erhält man, indem man 2-Äthoxyacrylsäurehalogenide bzw. 2-Äthoxy-1-alkyl-acrylsäurehalogenide mit Cyclopropyl-amidin-hydrochlorid in Gegenwart eines Säureakzeptors wie z.B. Triäthylamin und gegebenenfalls unter Verwendung eines Verdünnungsmittels wie z.B. Acetonitril bei Temperaturen zwischen 0 und 50°C umsetzt.

Das auf diese Weise hergestellte 2-Cyclopropyl-4-hydroxy-pyrimidin kann durch Umsetzung mit Halogenen



BAD ORIGINAL

wie Chlor oder Brom, gegebenenfalls in Gegenwart eines Säureakzeptors wie z.B. Kaliumhydroxid und gegebenenfalls unter Verwendung eines Verdünnungsmittels wie z.B. Wasser, bei Temperaturen zwischen o und 5o°C in 5-Chlor- bzw. 5-Brom-2-cyclopropyl-4-hydroxypyrimidin umgewandelt werden.

Das Verfahren zur Herstellung der erfindungsgemäßen 2-Cyclopropyl-pyrimidin(4)yl-(thiono)(thiol)-phosphor-(phosphon)-säureester bzw. -esteramide wird bevorzugt unter Verwendung geeigneter Lösungs- oder Verdünnungsmittel durchgeführt. Als solche kommen praktisch alle inerten organischen Solventien infrage. Hierzu gehören insbesondere aliphatische und aromatische, gegebenenfalls chlorierte Kohlenwasserstoffe, wie Benzin, Benzol, Toluol, Xylol, Methylenchlorid, Chloroform, Tetrachlorkohlenstoff, Chlorbenzol und o-Dichlorbenzol, Äther wie Diäthyl- und Dibutyläther, Tetrahydrofuran und Dioxan, Ketone wie Aceton, Methyläthyl-, Methylisopropyl- und Methylisobutylketon sowie Nitrile wie Acetonitril und Propionitril.

Als Säureakzeptoren können alle üblichen Säurebindemittel Verwendung finden. Besonders bewährt haben sich Alkalicarbonate und -alkoholate, wie Natrium- und Kaliumcarbonat, Natrium- und Kaliummethylat bzw. -äthylat, ferner aliphatische, aromatische oder heterocyclische Amine, beispielsweise Triäthylamin, Trimethylamin, Dimethylanilin, Dimethylbenzylamin und Pyridin.

Die Reaktionstemperatur kann innerhalb eines größeren Bereichs variiert werden. Im allgemeinen arbeitet man zwischen

-lo und loo°C, vorzugsweise bei 0 bis 8o°C. Das erfindungsgemäße Verfahren wird im allgemeinen bei Normaldruck durchgeführt.

Zur Durchführung des erfindungsgemäßen Verfahrens werden die Ausgangsstoffe gewöhnlich in äquimolaren Mengen eingesetzt. Ein Überschuß der einen oder anderen Reaktionskomponente bringt keine wesentlichen Vorteile. Die Umsetzung wird im allgemeinen in einem geeigneten Verdünnungsmittel in Gegenwart eines Säureakzeptors durchgeführt und das Reaktionsgemisch wird mehrere Stunden bei der erforderlichen Temperatur gerührt. Danach gibt man ein organisches Lösungsmittel, z.B. Toluol, zu und arbeitet die organische Phase wie üblich durch Waschen, Trocknen und Abdestillieren des Lösungsmittels auf.

Die neuen Verbindungen fallen in Form von Ölen an, die sich zum Teil nicht unzersetzt destillieren lassen, jedoch durch sogenanntes "Andestillieren", d.h. durch längeres Erhitzen unter vermindertem Druck auf mäßig erhöhte Temperaturen von den letzten flüchtigen Anteilen befreit und auf diese Weise gereinigt werden. Zu ihrer Charakterisierung dient der Brechungsindex.

Die erfindungsgemäßen 2-Cyclopropyl-pyrimidin(4)yl-(thiono)-(thiol)-phosphor(phosphon)-säureester bzw. -esteramide zeichnen sich durch eine hervorragende Wirkung als Schädlingsbekämpfungsmittel, insbesondere durch insektizide, akarizide und nematizide Wirksamkeit aus. Sie wirken gegen Pflanzen-, Hygiene- und Vorratsschädlinge sowie gegen Ektoparasiten. Bei geringer Phytotoxizität besitzen sie eine gute Wirkung gegen saugende und fressende Insekten und Milben.

Aus diesem Grunde können die erfindungsgemäßen Verbindungen mit Erfolg im Pflanzenschutz sowie auf dem Hygiene- und Vorratsschutz- und veterinär-medizinischen Sektor als Schädlingsbekämpfungsmittel eingesetzt werden.

Die Wirkstoffe eignen sich bei guter Pflanzenverträglichkeit und günstiger Warmblütertoxizität zur Bekämpfung von tierischen Schädlingen, insbesondere Insekten, Spinnentieren und Nematoden, die in der Landwirtschaft, in Forsten, im Vorrats- und Materialschutz sowie auf dem Hyginesektor vorkommen. Sie sind gegen normal sensible und resistente Arten sowie gegen alle oder einzelne Entwicklungsstadien wirksam. Zu den oben erwähnten Schädlingen gehören:

Aus der Ordnung der Isopoda z.B. Oniscus asellus, Armadillidium vulgare, Porcellio scaber.
Aus der Ordnung der Diplopoda z.B. Blaniulus guttulatus.
Aus der Ordnung der Chilopoda z.B. Geophilus carpophaus, Scutigera spec.
Aus der Ordnung der Symphyla z.B. Scutigerella immaculata.
Aus der Ordnung der Thysanura z.B. Lepisma saccharina.
Aus der Ordnung der Collembola z.B. Onychiurus armatus.
Aus der Ordnung der Orthoptera z.B. Blatta orientalis, Periplaneta americana, Leucophaea maderae, Blattella germanica, Acheta domesticus, Gryllotalpa spp., Locusta migratoria migratorioides, Melanoplus differentialis, Schistocerca gregaria.
Aus der Ordnung der Dermaptera z.B. Forficula auricularia.
Aus der Ordnung der Isoptera z.B. Reticulitermes spp..
Aus der Ordnung der Anoplura z.B. Phylloxera vastatrix, Pemphigus spp., Pediculus humanus corporis, Haematopinus spp., Linognathus spp.
Aus der Ordnung der Mallophaga z.B. Trichodectes spp., Damalinea spp.
Aus der Ordnung der Thysanoptera z.B. Hercinothrips fermoralis, Thrips tabaci.

Aus der Ordnung der Heteroptera z.B. Eurygaster spp., Dysdercus intermedius, Piesma quadrata, Cimex lectularius, Rhodnius prolixus, Triatoma spp.

Aus der Ordnung der Homoptera z.B. Aleurodes brassicae, Bemisia tabaci, Trialeurodes vaporariorum, Aphis gossypii, Brevicoryne brassicae, Cryptomyzus ribis, Doralis fabae, Doralis pomi, Eriosoma lanigerum, Hyalopterus arundinis, Macrosiphum avenae, Myzus spp.,Phorodon humuli, Rhopalosiphum padi, Empoasca spp., Euscelis bilobatus, Nephotettix cincticeps, Lecanium corni, Saissetia oleae, Laodelphax striatellus, Nilaparvata lugens, Aonidiella aurantii, Aspidiotus hederae, Pseudococcus spp. Psylla spp.

Aus der Ordnung der Lepidoptera z.B. Pectinophora gossypiella, Bupalus piniarius, Cheimatobia brumata, Lithocolletis blancardella, Hyponomeuta padella, Plutella maculipennis, Malacosoma neustria, Euproctis chrysorrhoea, Lymantria spp. Bucculatrix thurberiella, Phyllocnistis citrella, Agrotis spp., Euxoa spp., Feltia spp. Earias insulana, Heliothis spp., Laphygma exigua, Mamestra brassicae, Panolis flammea, Prodenia litura, Spodoptera spp., Trichoplusia ni, Carpocapsa pomonella, Pieris spp., Chilo spp., Pyrausta nubilalis, Ephestia kuehniella, Galleria mellonella, Cacoecia podana, Capua reticulana, Choristoneura fumiferana, Clysia ambiguella, Homona magnanima, Tortrix viridana.

Aus der Ordnung der Coleoptera z.B. Anobium punctatum, Rhizopertha dominica, Bruchidius obtectus, Acanthoscelides obtectus, Hylotrupes bajulus, Agelastica alni, Leptinotarsa decemlineata, Phaedon cochleariae, Diabrotica spp., Psylliodes chrysocephala, Epilachna varivestis, Atomaria spp., Oryzaephilus surinamensis, Anthonomus spp., Sitophilus spp., Otiorrhynchus sulcatus, Cosmopolites sordidus, Ceuthorrhynchus assimilis, Hypera postica, Dermestes spp.,

Trogoderma spp., Anthrenus spp., Attagenus spp., Lyctus spp., Meligethes aeneus, Ptinus spp., Niptus hololeucus, Gibbium psylloides, Tribolium spp., Tenebrio molitor, Agriotes spp., Conoderus spp., Melolontha melolontha, Amphimallon solstitialis, Costelytra zealandica.
Aus der Ordnung der Hymenoptera z.B. Diprion spp., Hoplocampa spp., Lasius spp., Monomorium pharaonis, Vespa spp.
Aus der Ordnung der Diptera z.B. Aedes spp., Anopheles spp., Culex spp., Drosophila melanogaster, Musca spp., Fannia spp., Calliphora erythrocephala, Lucilia spp., Chrysomyia spp., Cuterebra spp., Gastrophilus spp., Hyppobosca spp., Stomoxys spp., Oestrus spp., Hypoderma spp., Tabanus spp., Tannia spp., Bibio hortulanus, Oscinella frit, Phorbia spp., Pegomyia hyoscyami, Ceratitis capitata, Dacus oleae, Tipula paludosa.

Aus der Ordnung der Siphonaptera z.B. Xenopsylla cheopis, Ceratophyllus spp..
Aus der Ordnung der Arachnida z.B. Scorpio maurus, Latrodectus mactans.
Aus der Ordnung der Acarina z.B. Acarus siro, Argas spp., Ornithodoros spp., Dermanyssus gallinae, Eriophyes ribis, Phyllocoptruta oleivora, Boophilus spp., Rhipicephalus spp., Amblyomma spp., Hyalomma spp., Ixodes spp., Psoroptes spp., Chorioptes spp., Sarcoptes spp., Tarsonemus spp., Bryobia praetiosa, Panonychus spp., Tetranychus spp..

Zu den pflanzenparasitären Nematoden gehören Pratylenchus spp., Radopholus similis, Ditylenchus dipsaci, Tylenchulus semipenetrans, Heterodera spp., Meloidogyne spp., Aphelenchoides spp., Longidorus spp., Xiphinema spp., Trichodorus spp..

Die Wirkstoffe können in die üblichen Formulierungen übergeführt werden, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chloräthylene oder Methylenchlorid, aliphatische Kohlenwasserstoffe, wie Cyclohexan oder Paraffine, z.B. Erdölfraktionen, Alkohole, wie Butanol oder Glycol sowie deren Äther und Ester, Ketone, wie Aceton, Methyläthylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgas, wie Halogenkohlenwasserstoffe sowie Butan, Propan, Stickstoff

und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyäthylen-Fettsäure-Ester, Polyoxyäthylen-Fettalkohol-Äther, z.B. Alkylarylpolyglykol-äther, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat.

Es können Farbstoffe wie anorganische Pigmente, z.B. Eisenoxid, Titanoxid, Ferrocyanblau und organische Farbstoffe, wie Alizarin-, Azol-Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die Anwendung der erfindungsgemäßen Wirkstoffe erfolgt in Form ihrer handelsüblichen Formulierungen und/oder den aus diesen Formulierungen bereiteten Anwendungsformen.

Der Wirkstoffgehalt der aus den handelsüblichen Formulierungen bereiteten Anwendungsformen kann in weiten Bereichen variieren. Die Wirkstoffkonzentration der Anwendungsformen kann von 0,0000001 bis zu 100 Gew.-% Wirkstoff, vorzugsweise zwischen 0,01 und 10 Gew.-% liegen.

Die Anwendung geschieht in einer den Anwendungsformen angepaßten üblichen Weise.

Bei der Anwendung gegen Hygiene- und Vorratsschädlinge zeichnen sich die Wirkstoffe durch eine hervorragende Residualwirkung auf Holz und Ton sowie durch eine gute Alkalistabilität auf gekälkten Unterlagen aus.

Die Anwendung der erfindungsgemäßen Wirkstoffe geschieht im Veterinärsektor in bekannter Weise, wie durch orale Anwendung in Form von beispielsweise Tabletten, Kapseln, Tränken, Granulaten, durch dermale Anwendung in Form beispielsweise des Tauchens (Dippen), Sprühens (Sprayen), Aufgießens (pour-on and spot-on) und des Einpuders sowie durch parenterale Anwendung in Form beispielsweise der Injektion.

## Beispiel A

Drosophila-Test

Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

1 $cm^3$ der Wirkstoffzubereitung wird auf eine Filterpapierscheibe (7 cm Durchmesser) aufpipettiert. Man legt diese naß auf die Öffnung eines Glasgefäßes, in dem sich 50 Taufliegen (Drosophila melanogaster) befinden und bedeckt es mit einer Glasplatte.

Nach der gewünschten Zeit bestimmt man die Abtötung in %. Dabei bedeutet 100 %, daß alle Fliegen abgetötet wurden; 0 % bedeutet, daß keine Fliegen abgetötet wurden.

Bei diesem Test zeigen z.B. die folgenden Verbindungen der Herstellungsbeispiele überlegene Wirksamkeit gegenüber dem Stand der Technik: 2, 3, 8, 9 und 10.

Beispiel B

Plutella-Test

Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Kohlblätter (Brassica oleracea) werden durch Tauchen in die Wirkstoffzubereitung der gewünschten Konzentration behandelt und mit Raupen der Kohlschabe (Plutella maculipennis) besetzt, solange die Blätter noch feucht sind.

Nach der gewünschten Zeit wird die Abtötung in % bestimmt. Dabei bedeutet 100 %, daß alle Raupen abgetötet wurden; 0 % bedeutet, daß keine Raupen abgetötet wurden.

Bei diesem Test zeigen z.B. die folgenden Verbindungen der Herstellungsbeispiele überlegene Wirksamkeit gegenüber dem Stand der Technik: 1, 2, 3, 4, 6, 8, 9 und 10.

Beispiel C

Tetranychus-Test (resistent)

Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel und der angegebenen Menge Emulgator und verdünnt das Konzentrat mit Wasser auf die gewünschte Konzentration.

Bohnenpflanzen (Phaseolus vulgaris),die stark von allen Entwicklungsstadien der gemeinen Spinnmilbe oder Bohnenspinnmilbe (Tetranychus urticae) befallen sind, werden durch Tauchen in die Wirkstoffzubereitung der gewünschten Konzentration behandelt.

Nach der gewünschten Zeit wird die Abtötung in % bestimmt. Dabei bedeutet 100 %, daß alle Spinnmilben abgetötet wurden; 0 % bedeutet, daß keine Spinnmilben abgetötet wurden.

Bei diesem Test zeigen z.B. die folgenden Verbindungen der Herstellungsbeispiele überlegene Wirksamkeit gegenüber dem Stand der Technik: 1, 2, 3, 6, 8, 9 und 10.

Beispiel D

$LT_{100}$-Test für Dipteren

Testtiere: Aëdes aegypti
Zahl der Testtiere: 25
Lösungsmittel: Aceton

2 Gewichtsteile Wirkstoff werden in 1000 Volumenteilen Lösungsmittel aufgenommen. Die so erhaltene Lösung wird mit weiterem Lösungsmittel auf die gewünschten geringeren Konzentrationen verdünnt.

2,5 ml Wirkstofflösung werden in eine Petrischale pipettiert. Auf dem Boden der Petrischale befindet sich ein Filterpapier mit einem Durchmesser von etwa 9,5 cm. Die Petrischale bleibt so lange offen stehen, bis das Lösungsmittel vollständig verdunstet ist. Je nach Konzentration der Wirkstofflösung ist die Menge Wirkstoff pro $m^2$ Filterpapier verschieden hoch. Anschließend gibt man die angegebene Anzahl der Testtiere in die Petrischale und bedeckt sie mit einem Glasdeckel.

Der Zustand der Testtiere wird laufend kontrolliert. Es wird diejenige Zeit ermittelt, welche für einen 100 %igen knock down-Effekt notwendig ist.

Bei diesem Test zeigen z.B. die folgenden Verbindungen der Herstellungsbeispiele überlegene Wirkung gegenüber dem Stand der Technik: 1, 2 und 3.

Beispiel E

Grenzkonzentrations-Test / Nematoden

Testnematode: Meloidogyne incognita
Lösungsmittel: 3 Gewichtsteile Aceton
Emulgator: 1 Gewichtsteil Alkylarylpolyglykoläther

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Die Wirkstoffzubereitung wird innig mit Boden vermischt, der mit den Testnematoden stark verseucht ist. Dabei spielt die Konzentration des Wirkstoffs in der Zubereitung praktisch keine Rolle, entscheidend ist allein die Wirkstoffmenge pro Volumeneinheit Boden, welche in ppm angegeben wird. Man füllt den behandelten Boden in Töpfe, sät Salat ein und hält die Töpfe bei einer Gewächshaus-Temperatur von 27°C.

Nach vier Wochen werden die Salatwurzeln auf Nematodenbefall (Wurzelgallen) untersucht und der Wirkungsgrad des Wirkstoffs in % bestimmt. Der Wirkungsgrad ist 100 %, wenn der Befall vollständig vermieden wird, er ist 0 %, wenn der Befall genau so hoch ist wie bei den Kontrollpflanzen in unbehandeltem, aber in gleicher Weise verseuchtem Boden.

Bei diesem Test zeigen z.B. die folgenden Verbindungen der Herstellungsbeispiele überlegene Wirksamkeit gegenüber dem Stand der Technik: 1, 2 und 6.

Beispiel F

Test mit parasitierenden Fliegenlarven (Lucilia cuprina res.)

Emulgator: 80 Gewichtsteile Alkylarylpolyglykoläther

Zur Herstellung einer zweckmäßigen Wirkstoffzubereitung vermischt man 20 Gew.-Teile der betreffenden aktiven Substanz mit der angegebenen Menge des Emulgators und verdünnt das so erhaltene Gemisch mit Wasser auf die gewünschte Konzentration. Etwa 20 Fliegenlarven (Lucilia cuprina) werden in ein mit Wattestopfen entsprechender Größe beschicktes Teströhrchen gebracht, welches ca. 3 ml einer 20 %igen Eigelbpulver-Suspension in Wasser enthält. Auf diese Eigelbpulver-Suspension werden 0,5 ml der Wirkstoffzubereitung gebracht. Nach 24 Stunden wird der Abtötungsgrad bestimmt.

Bei diesem Test zeigen z.B. die folgenden Verbindungen der Herstellungsbeispiele überlegene Wirkung gegenüber dem Stand der Technik: 1, 2 und 3.

Beispiel G

Test mit parasitierenden adulten Rinderzecken (Boophilus microplus res.)

Lösungsmittel: Alkylarylpolyglykoläther

Zur Herstellung einer zweckmäßigen Wirkstoffzubereitung vermischt man die betreffende aktive Substanz mit dem angegebenen Lösungsmittel im Verhältnis 1:2 und verdünnt das so erhaltene Konzentrat mit Wasser auf die gewünschte Konzentration.

10 adulte Rinderzecken (b.microplus res.) werden 1 Minute in die zu testende Wirkstoffzubereitung getaucht. Nach Überführung in Plastikbecher und Aufbewahrung in einem klimatisierten Raum wird der Abtötungsgrad bestimmt.

Bei diesem Test zeigen z.B. die folgenden Verbindungen der Herstellungsbeispiele überlegene Wirkung gegenüber dem Stand der Technik: 3.

Herstellungsbeispiele

Beispiel 1:

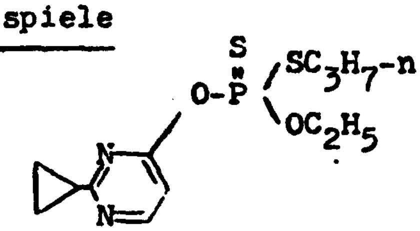

Zu einer Mischung von 13,6 g (o,1 Mol) 2-Cyclopropyl-4-hydroxy-pyrimidin, 2o,7 g (o,15 Mol) Kaliumcarbonat und 3oo ml Acetonitril tropft man 21,8 g (o,1 Mol) O-Äthyl-S-n-propyl-thionothiolphosphorsäureesterchlorid und läßt den Ansatz noch 3 Stunden bei 45-5o°C nachreagieren. Die Reaktionsmischung wird danach abgekühlt, in 5oo ml Toluol gegossen und mit Wasser gewaschen. Dann wird die organische Phase über Natriumsulfat getrocknet, unter vermindertem Druck vom Lösungsmittel befreit und der Rückstand andestilliert. Man erhält 22,8 g (72 % der Theorie) O-Äthyl-S-n-propyl-O-(2-cyclopropyl-pyrimidin(4)yl)-thionothiolphosphorsäureester in Form eines gelben Öles mit dem Brechungsindex $n_D^{22}$: 1,5482.

In analoger Weise wie in Beispiel 1 werden die folgenden Verbindungen der Formel

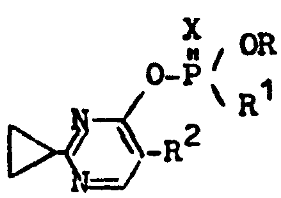

(I)

erhalten:

| Beispiel Nr. | R | $R^1$ | $R^2$ | X | Ausbeute (% der Theoric) | Brechungsindex: |
|---|---|---|---|---|---|---|
| 2 | $C_2H_5$ | $OC_2H_5$ | H | S | 76 | $n_D^{22}$:1,5152 |
| 3 | $C_2H_5$ | $CH_3$ | H | S | 68 | $n_D^{22}$:1,5373 |
| 4 | $C_2H_5$ | 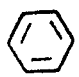 | H | S | 68 | $n_D^{22}$:1,5812 |
| 5 | $CH_3$ | $OCH_3$ | H | S | 90 | $n_D^{20}$: 1,5249 |
| 6 | $C_2H_5$ | $NH-C_3H_7$-iso | H | S | 54 | $n_D^{23}$:1,5268 |
| 7 | $C_3H_7$-n | $OC_2H_5$ | H | S | 91 | $n_D^{20}$:1,5075 |
| 8 | $CH_3$ | $OC_3H_7$-n | H | S | 77 | $n_D^{24}$:1,5264 |
| 9 | $C_2H_5$ | $OCH_3$ | H | S | 71 | $n_D^{24}$:1,5293 |
| 1o | $C_2H_5$ | $OC_2H_5$ | Br | S | 6o | $n_D^{24}$:1,5417 |
| 11 | $C_2H_5$ | 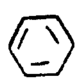 | Br | S | | |
| 12 | $C_2H_5$ | $OC_2H_5$ | Cl | S | | |

| Beispiel Nr. | R | $R^1$ | $R^2$ | X | Ausbeute (% der Theorie) | Brechungsindex: |
|---|---|---|---|---|---|---|
| 13 | $C_2H_5$ | 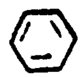 | Cl | S | | |
| 14 | $C_2H_5$ | $OC_2H_5$ | $CH_3$ | S | | |
| 15 | $C_2H_5$ | 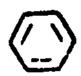 | $CH_3$ | S | | |
| 16 | $C_2H_5$ | $SC_3H_7$-n | $CH_3$ | S | | |
| 17 | $C_2H_5$ | $OC_2H_5$ | H | O | | |
| 18 | $C_2H_5$ | $OC_2H_5$ | $C_3H_7$-iso | S | | |
| 19 | $C_2H_5$ | $OC_2H_5$ | $C_2H_5$ | S | | |

Die als Ausgangsmaterialien zu verwendenden 2-Cyclopropyl-4-hydroxy-pyrimidine können z.B. wie folgt hergestellt werden:

Beispiel a: 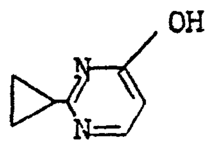

Zu einer Mischung aus 6o,3 g (o,5 Mol) Cyclopropionamidin-Hydrochlorid, 1o1 g (1 Mol) Triäthylamin und 5oo ml Acetonitril tropft man bei 15-2o°C 67,5 g (o,5 Mol) 2-Äthoxyacrylsäurechlorid und rührt anschließend 3 Stunden bei Raumtemperatur nach. Dann destilliert man das Lösungsmittel im Vakuum ab, verrührt den Rückstand 2 mal mit je 5oo ml Essigsäureäthylester, filtriert vom Salz ab und dampft das Filtrat im Vakuum ein. Man erhält so 42,9 g (64 % der Theorie) 2-Cyclopropyl-4-hydroxy-pyrimidin in Form farbloser Kristalle mit dem Schmelzpunkt 148°C.

Beispiel b: 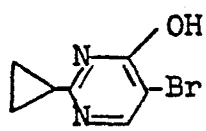

Zu einer Lösung von 5,6 g (o,1 Mol) Kaliumhydroxyd in 1oo ml Wasser gibt man 13,6 g (o,1 Mol) 2-Cyclopropyl-4-hydroxy-pyrimidin. Unter schwacher Kühlung tropft man dann bei 15-2o°C 16 g (o,1 Mol) Brom zu und rührt das Gemisch 1 Stunde bei Raumtemperatur nach. Das ausgefallene Produkt wird abgesaugt und mit Wasser nachgewaschen. Man erhält auf diese Weise

15 g (7o % der Theorie) 2-Cyclopropyl-4-hydroxy-5-brom-pyrimidin in Form farbloser Kristalle mit dem Schmelzpunkt 2oo°C.

Analog einem der Beispiele a oder b können die folgenden Verbindungen der Formel

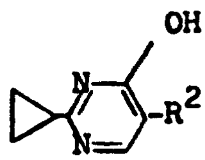

hergestellt werden:

| Beispiel | $R^2$ |
|---|---|
| c | $CH_3$ |
| d | Cl |
| e | $C_3H_7$-iso |
| f | $C_2H_5$ |

## Patentansprüche

1. 2-Cyclopropyl-pyrimidin(4)yl-(thiono)-(thiol)-phosphor(phosphon)-säureester bzw. -esteramide der Formel

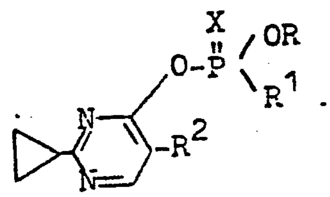

(I)

in welcher

R für Alkyl steht,

$R^1$ für Alkyl, Alkoxy, Alkylthio, Alkylamino oder Phenyl steht,

$R^2$ für Wasserstoff, Alkyl oder Halogen steht und

X für Sauerstoff oder Schwefel steht.

2. Verfahren zur Herstellung von 2-Cyclopropyl-pyrimidin(4)yl-(thiono)-(thiol)-phosphor(phosphon)-säureestern bzw. esteramiden der Formel (I), dadurch gekennzeichnet, daß man (Thiono)(Thiol)Phosphor(phosphon)-säureesterhalogenide bzw. (Thiono)-Phosphorsäureesteramidhalogenide der Formel

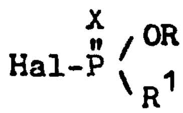 (II)

in welcher

R, $R^1$ und X die oben angegebene Bedeutung haben und

Hal für Chlor oder Brom steht,

mit 2-Cyclopropyl-4-hydroxy-pyrimidinen der Formel

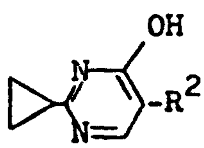 (III)

in welcher

$R^2$ die oben angegebene Bedeutung hat,

gegebenenfalls in Gegenwart eines Säureakzeptors und gegebenenfalls in Gegenwart eines Verdünnungsmittels umsetzt.

3. Schädlingsbekämpfungsmittel, gekennzeichnet durch einen Gehalt an Verbindungen gemäß Anspruch 1.

4. Insektizide, akarizide und nematizide Mittel nach Anspruch 3, gekennzeichnet durch einen Gehalt an Verbindungen gemäß Anspruch 1.

5. Verfahren zur Bekämpfung von Insekten, Milben und Nematoden, dadurch gekennzeichnet, daß man Verbindungen gemäß Anspruch 1 auf die genannten Schädlinge bzw. deren Lebensraum einwirken läßt.

6. Verwendung von Verbindungen gemäß Anspruch 1 zur Bekämpfung von Schädlingen.

7. Verfahren zur Herstellung von insektiziden, akariziden und nematiziden Mitteln, dadurch gekennzeichnet, daß man Verbindungen gemäß Anspruch 1 mit Streckmitteln und/oder oberflächenaktiven Mitteln vermischt.

Europäisches Patentamt

# EUROPÄISCHER RECHERCHENBERICHT

Nummer der Anmeldung
EP 79 102 329.4

| EINSCHLÄGIGE DOKUMENTE | | | KLASSIFIKATION DER ANMELDUNG (Int.Cl.³) |
|---|---|---|---|
| Kategorie | Kennzeichnung des Dokuments mit Angabe, soweit erforderlich, der maßgeblichen Teile | betrifft Anspruch | |
| P | DE - A1 - 2 737 401 (BAYER)<br>*Ansprüche 1 bis 6 *<br>-- | 2-7 | C 07 F 9/65<br>A 01 N 57/08<br>A 01 N 57/16<br>A 01 N 57/32 |
| A | DE - A - 2 412 903 (SANDOZ)<br>-- | | |
| A | FR - A - 2 168 183 (R. ARIES)<br>-- | | |
| A | US - A - 4 012 506 (CIBA-GEIGY)<br>---- | | |

RECHERCHIERTE SACHGEBIETE (Int. Cl.³)

A 01 N 57/08
A 01 N 57/16
A 01 N 57/32
C 07 F 9/65

KATEGORIE DER GENANNTEN DOKUMENTE

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Berlin | 07-11-1979 | KAPTEYN |